# EUROPÄISCHE PATENTANMELDUNG

(11) **EP 2 289 881 A1**
(43) Veröffentlichungstag der Anmeldung: **02.03.2011**
(21) Anmeldenummer: 09167393.9
(22) Anmeldetag: 06.08.2009
(51) Int. Cl.: C07D 239/70, C07D 403/12

(54) **Verfahren zur stereoselektiven Synthese bicyclischer Heterocyclen**

(71) Anmelder: Boehringer Ingelheim International GmbH, 55216 Ingelheim am Rhein (DE)
(72) Erfinder: Die Erfindernennung liegt noch nicht vor
(74) Vertreter: Hammann, Heinz

(57) **Zusammenfassung**

Die vorliegende Erfindung betrifft ein Verfahren zur stereoselektiven Herstellung von Verbindungen der Formeln **(IA)** und **(IB)** 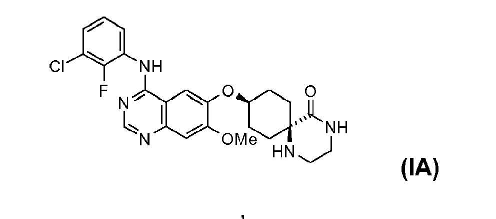 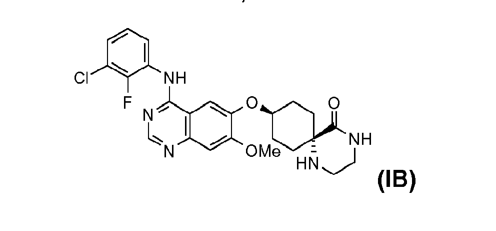 und deren Salze, insbesondere deren physiologisch verträgliche Salze mit anorganischen oder organischen Säuren und Basen, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege.

## Beschreibung

Gegenstand der vorliegenden Erfindung sind Verfahren zur stereoselektiven Herstellung von Verbindungen der Formeln **(IA)** und **(IB)** 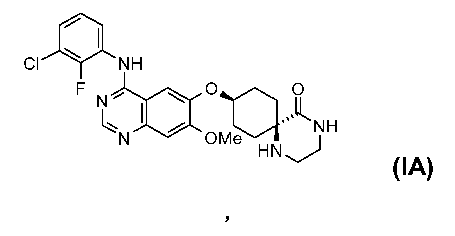 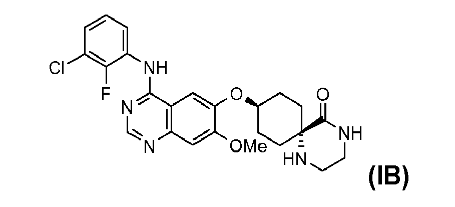 und deren Salze, insbesondere deren physiologisch verträgliche Salze mit anorganischen oder organischen Säuren und Basen, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege.

### Hintergrund der Erfindung

Chinazolinderivate sind aus dem Stand der Technik als Wirkstoffe beispielsweise zur Behandlung von Tumorerkrankungen als auch von Erkrankungen der Lunge und der Atemwege bekannt. Verfahren zur Herstellung von Chinazolinderivaten werden in WO03082831 beschrieben.

Es ist die Aufgabe der vorliegenden Erfindung ein stereoselektives Verfahren zur Herstellung der erfindungsgemäßen Chinazolinderivate bereitzustellen.

### Beschreibung der Erfindung

Die vorliegende Erfindung löst die vorstehend genannte Aufgabe über das im Folgenden beschriebene Syntheseverfahren.

Gegenstand der Erfindung ist somit ein Verfahren zur stereoselektiven Herstellung der Verbindung der Formel **(IA)** 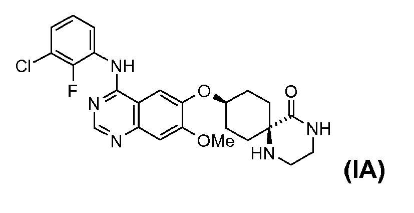 gegebenenfalls in Form ihrer Tautomeren, sowie gegebenenfalls ihrer pharmakologisch unbedenklichen Säureadditionssalze,
**dadurch gekennzeichnet, dass** das Verfahren die Reaktionsschritte **(A), (B), (V), (X), (R), (S)** und **(T)** umfasst, wobei
**(A)** die Umsetzung von 1,4.cyclohexandion-mono-ethylenketal zu einer Verbindung der Formel **(1)** 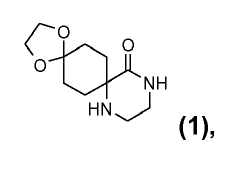
**(B)** die Umsetzung einer Verbindung der Formel **(1)** zu der Verbindung der Formel **(2)** 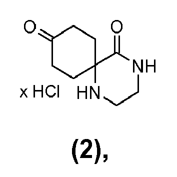
**(V)** die Umsetzung einer Verbindung der Formel **(2)** mit Chlorameisensäurebenzylester zu der Verbindung der Formel **(19)** 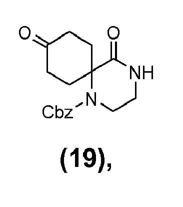
**(X)** die Umsetzung einer Verbindung der Formel **(19)** zu der Verbindung der Formel **(18)** 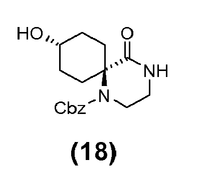
**(R)** die Umsetzung einer Verbindung der Formel **(18)** mit einer Verbindung der Formel **(23)** 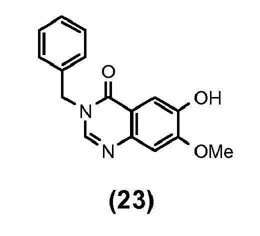 zu einer Verbindung der Formel **(21)** 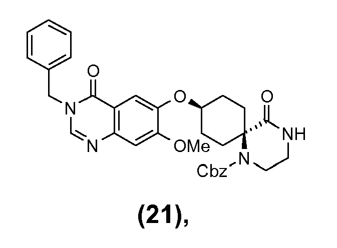
**(S)** die Abspaltung des Benzylrestes der Verbindung der Formel **(21)** zu einer Verbindung der Formel **(22)** 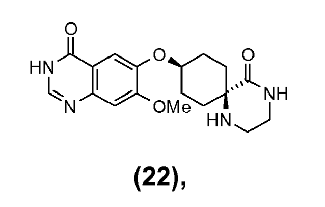 und
**(T)** die Chlorierung der Verbindung der Formel **(22)** und anschließende Umsetzung mit 3-Chlor-2-Fluoranilin
bedeutet,
wobei die Schritte **(A)** bis **(T)** in der genannten Reihenfolge nacheinander erfolgen.

Bevorzugt ist ein Verfahren zur stereoselektiven Herstellung von Verbindungen der Formel **(IA), dadurch gekennzeichnet, dass** das Verfahren aus den Verfahrensschritten **(R), (S)** und **(T)** besteht.

Ferner bevorzugt ist ein Verfahren zur stereoselektiven Herstellung einer Verbindung der Formel **(18), dadurch gekennzeichnet, dass** das Verfahren aus den Verfahrensschritten **(A), (B), (V)** und **(X)** besteht.

Ein weiterer Gegenstand der Erfindung ist ein Verfahren zur stereoselektiven Herstellung der Verbindung der Formel **(IB)** 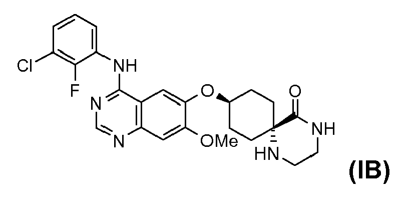 gegebenenfalls in Form ihrer Tautomeren, sowie gegebenenfalls ihrer pharmakologisch unbedenklichen Säureadditionssalze,
**dadurch gekennzeichnet, dass** das Verfahren die Reaktionsschritte **(A), (B), (Z), (H), (P), (Q), (M), (N)** und **(O)** umfasst, wobei
**(A)** die Umsetzung von 1,4.Cyclohexandion-mono-ethylenketal zu einer Verbindung der Formel **(1)** 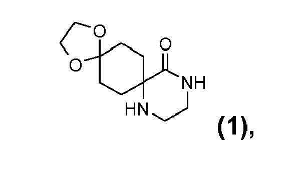
**(B)** die Umsetzung einer Verbindung der Formel **(1)** zu der Verbindung der Formel **(2)** 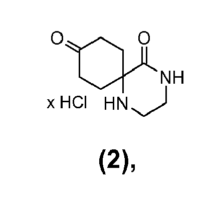
**(Z)** die Umsetzung einer Verbindung der Formel **(2)** zu der Verbindung der Formel **(16)** 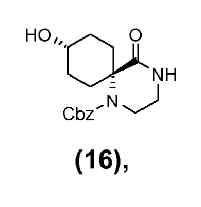
**(H)** die Umsetzung einer Verbindung der Formel **(16)** zu der Verbindung der Formel **(6)** 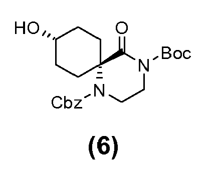
**(P)** die Umsetzung einer Verbindung der Formel **(6)** mit einer Verbindung der Formel **(23)** 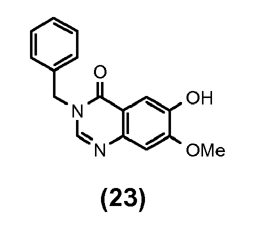 zu einer Verbindung der Formel **(7)** 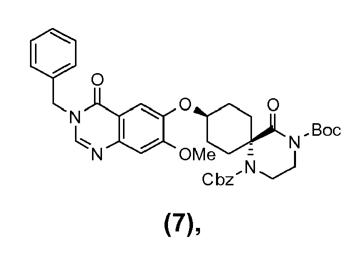
**(Q +M)** die Abspaltung der Schutzgruppen der Verbindung der Formel **(7)** zu einer Verbindung der Formel **(12)** 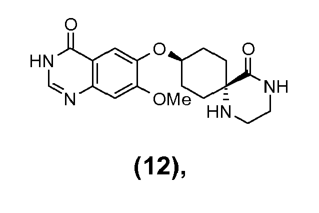 und
**(N + O)** die Chlorierung der Verbindung der Formel **(12)** und anschließende Umsetzung mit 3-Chlor-2-Fluoranilin
bedeutet,
wobei die Schritte **(A)** bis **(O)** in der genannten Reihenfolge nacheinander erfolgen.

Ein weiterer Gegenstand der Erfindung ist ein Verfahren zur stereoselektiven Herstellung einer Verbindung der Formel **(1B), dadurch gekennzeichnet, dass** in dem Verfahren die Verfahrensschritte **[(Z), (H)]** durch die Verfahrensschritte **[(C ), (D), (E), (F)]** ersetzt werden, wobei
**(C)** die Umsetzung einer Verbindung der Formel **(2)** zu der Verbindung der Formel **(3)** 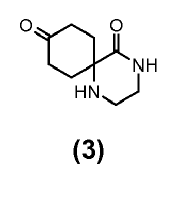
**(D)** die Umsetzung einer Verbindung der Formel **(3)** zu der Verbindung der Formel **(4)** 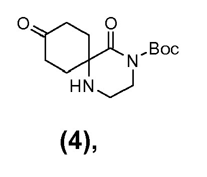
**(E)** die Umsetzung einer Verbindung der Formel **(4)** zu der Verbindung der Formel **(5)** 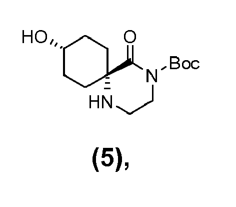 und
**(F)** die Umsetzung einer Verbindung der Formel **(5)** zu der Verbindung der Formel **(6)** 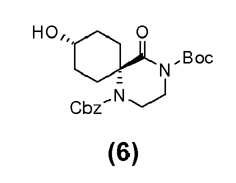 bedeuten,
wobei die Schritte **(C)** bis **(F)** in der genannten Reihenfolge nacheinander erfolgen.

Ein weiterer Gegenstand der Erfindung ist ein Verfahren zur stereoselektiven Herstellung einer Verbindung der Formel **(1B), dadurch gekennzeichnet, dass** in dem Verfahren die Verfahrensschritte **[(P), (Q), (M)]** durch die Verfahrensschritte **[(I**), **(J), (K), (L)]** ersetzt werden, wobei
**(I)** die Umsetzung einer Verbindung der Formel **(6)** mit einer Verbindung der Formel **(15)** 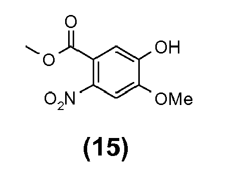 zu der Verbindung der Formel **(9)** 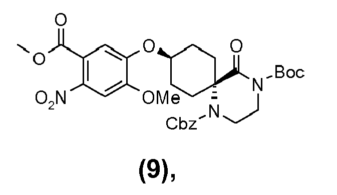
**(J + K)** die Abspaltung der Schutzgruppen und hydrogenolytische Reduktion einer Verbindung der Formel **(9)** zu der Verbindung der Formel **(11)** 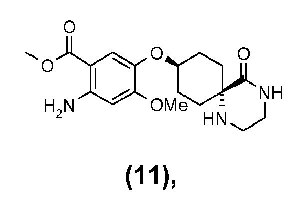 und
**(L)** die Umsetzung einer Verbindung der Formel **(11)** zu der Verbindung der Formel **(12)** 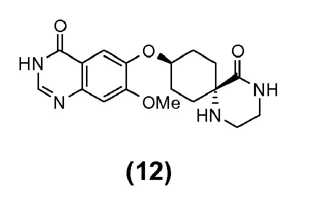 bedeuten,
   wobei die Schritte **(I)** bis **(L)** in der genannten Reihenfolge nacheinander erfolgen.
   Ein weiterer Gegenstand der Erfindung ist die Verbindung der Formel 1A, sowie deren pharmakologisch verträglichen Salze, Hydrate, Solvate und Co-Kristalle.
   Ein weiterer Gegenstand der Erfindung ist die Verbindung der Formel 1B, sowie deren pharmakologisch verträglichen Salze, Hydrate, Solvate und Co-Kristalle.
   Ein weiterer Gegenstand der Erfindung ist die Verbindung der Formel 18, sowie deren pharmakologisch verträglichen Salze, Hydrate, Solvate und Co-Kristalle.
   Ein weiterer Gegenstand der Erfindung ist die Verbindung der Formel 22, sowie deren pharmakologisch verträglichen Salze, Hydrate, Solvate und Co-Kristalle.
   Ein weiterer Gegenstand der Erfindung ist die Verbindung der Formel 13, sowie deren pharmakologisch verträglichen Salze, Hydrate, Solvate und Co-Kristalle.
   Ein weiterer Gegenstand der Erfindung ist die Verbindung der Formel 16., sowie deren pharmakologisch verträglichen Salze, Hydrate, Solvate und Co-Kristalle.
   Ein weiterer Gegenstand der Erfindung ist die Verbindung der Formel 6, sowie deren pharmakologisch verträglichen Salze, Hydrate, Solvate und Co-Kristalle.

Unter Co-Kristallen sind im Rahmen der vorliegenden Erfindung Molekülkomplexe, die zwei oder mehr verschiedene Moleküle in demselben Kristallgitter enthalten zu verstehen (Crystal Growth & Design, 2009, Vol. 9, No. 6, 2950-2967; Stahly, G. P. Cryst. Growth Des. 2007, 7, 1007-1026), insbesondere Co-Kristalle, die gebildet werden zwischen einem molekularen oder ionischen pharmazeutischen Wirkstoffmolekül und einem Co-Kristall-Bildner, der bei Raumtemperatur als Feststoff vorliegt (Jones, W.; Motherwell, W. D.; Trask, A. V. MRS Bull. 2006, 341, 875-879; Vishweshwar, P.; McMahon, J. A.; Bis, J. A.; Zaworotko, M. J. J. Pharm. Sci. 2006, 95, 499-516).

Weiterhin Insbesondere bevorzugt ist ein Verfahren, worin ein Chlorierungsmittel ausgewählt aus der Gruppe bestehend aus, Thionylchlorid, Phosphoroxychlorid, ,einer N-Chlorsuccinimid/ Triphenylphosphan - Kombination, einer Tetrachlorkohlenstoff/Triphenylphosphan-Kombination, eingesetzt wird.

Die erfindungsgemäßen Verbindungen können in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren, beispielsweise Chlor- oder Bromwasserstoffsäure, anorganischen Säuren, beispielsweise Phosphorsäure oder Schwefelsäure oder organischen Säuren, wie beispielsweise Oxal-, Fumar-, Diglycol-, Toluolsulfon-, Benzoe-, Bernstein-, Malein-, Salicyl-, Äpfel- oder Methansulfonsäure, vorliegen.

Der Einsatz folgender Lösungsmittel ausgewählt aus der jeweils aufgeführten Gruppe wird in den oben beschriebenen Verfahrenschritten bevorzugt:
In Verfahrensschritt
A: CH₂Cl₂, CHCl₃, THF (Tetrahydrofuran) und Dioxan
B: HOAc, H₂O, wässrige Lösungen folgender Lösungsmittel: EtOH, THF, iPrOH, MeOH, NMP (N-Methyl-2-pyrrolidon) und DMF (Dimethylformamid)
V: THF, Dioxan, NMP, Me-THFund ACN (Acetonitril)
X: THF/EtOH/H₂O und Dioxan/MeOH/H₂O
R: NMP, Dioxan, DMF, THF und CH₂Cl₂
S: EtOH/H₂O/HCl, HOAc und MeOH/H₂O/HCl
T: ACN und NMP
Z: wässrige NaOH und wässrige KOH, zusätzlich dazu EtOH, MeOH, THF
P: NMP, Dioxan, THF und CH₂Cl₂
Q: Dioxan, THF, NMP, CH₂Cl₂ und EtOH
M: HOAc/H₂O, HCl/EtOH und HCl/MeOH
N: Dioxan/ACN und THF/ACN
O: HCl/H₂O, NMP, Dioxan und THF
C: ACN, EtOH, MeOH, iPrOH, H₂O, THF und NMP
D: ACN, THF und NMP
E: H₂O, EtOH, THF und Dioxan
F: H₂O, THF und EtOH
G: H₂O/THF, THF, NMP, CH₂Cl₂ und Dioxan
I: NMP, THF, Dioxan, CH₂Cl₂, Toluol und DMF
J: Dioxan, THF, NMP, CH₂Cl₂ und EtOH
K: EtOH, MeOH, iPrOH, NMP und THF
L: nPrOH, EtOH, MeOH, NMP und ACN

Die oben beschriebenen Verfahrensschritte werden vorzugsweise in folgenden Temperaturbereichen durchgeführt:
In Verfahrensschritt:
A: vorzugsweise -15 bis 40 °C, besonders bevorzugt -10 bis 10 °C,
B: vorzugsweise 20 bis 75 °C, besonders bevorzugt 35 bis 55 °C,
V: vorzugsweise 0 bis 50 °C, besonders bevorzugt 10 bis 35 °C,
X: vorzugsweise 0 bis 60 °C, besonders bevorzugt 5 bis 35 °C,
R: vorzugsweise 5 bis 100 °C, besonders bevorzugt 15 bis 40 °C,
S: vorzugsweise 50 bis 80 °C, besonders bevorzugt 65 bis 80 °C,
T: vorzugsweise 10 bis 80 °C, besonders bevorzugt 15 bis 50 °C,
Z: vorzugsweise 0 bis 60 °C, besonders bevorzugt 10 bis 35 °C,
H: vorzugsweise 15 bis 60 °C, besonders bevorzugt 15 bis 30 °C,
P: vorzugsweise 10 bis 80 °C, besonders bevorzugt 15 bis 35 °C,
Q: vorzugsweise 0 bis 80 °C, besonders bevorzugt 50 bis 70 °C,
M: vorzugsweise 20 bis 90 °C, besonders bevorzugt 60 bis 80 °C,
N: vorzugsweise 15 bis 85 °C, besonders bevorzugt 70 bis 85 °C,
O: vorzugsweise 0 bis 80 °C, besonders bevorzugt 10 bis 50 °C,
C: vorzugsweise 0 bis 65 °C, besonders bevorzugt 15 bis 30 °C,
D: vorzugsweise 10 bis 80 °C, besonders bevorzugt 20 bis 40 °C,
E: vorzugsweise 0 bis 40 °C, besonders bevorzugt 0 bis 15 °C,
F: vorzugsweise 0 bis 45 °C, besonders bevorzugt 10 bis 25 °C,
G: vorzugsweise 0 bis 45 °C, besonders bevorzugt 10 bis 25 °C,
I: vorzugsweise 0 bis 50 °C, besonders bevorzugt 15 bis 30 °C,
J: vorzugsweise 0 bis 85°C, besonders bevorzugt 40 bis 70 °C,
K: vorzugsweise 10 bis 60 °C, besonders bevorzugt 15 bis 35 °C, und
L: vorzugsweise 60 bis 97 °C, besonders bevorzugt 85 bis 97 °C,

In Verfahrensschritten K ,M und S werden vorzugsweise Katalysatoren ausgewählt aus der Gruppe bestehend aus Pd/C, Pd(OH)₂ bevorzugt Pd/C, eingesetzt.

Es werden vorzugsweise Schutzgruppen ausgewählt aus der Gruppe bestehend aus

Benzyl, Cbz, Trifluoracetyl und Boc eingesetzt.

Die in obigen Formeln verwendete Abkürzung **Boc** bedeutet teriär butyl carbamat und **Cbz** bedeutet Benzyloxycarbonyl .

Schema 1 und 2 illustrieren die erfindungsgemäße Synthese. Sämtliche

Verbindungen sind in Form ihrer Basen dargestellt.

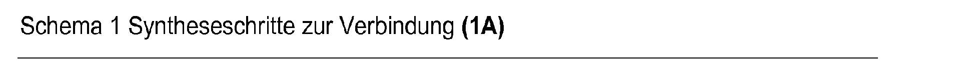

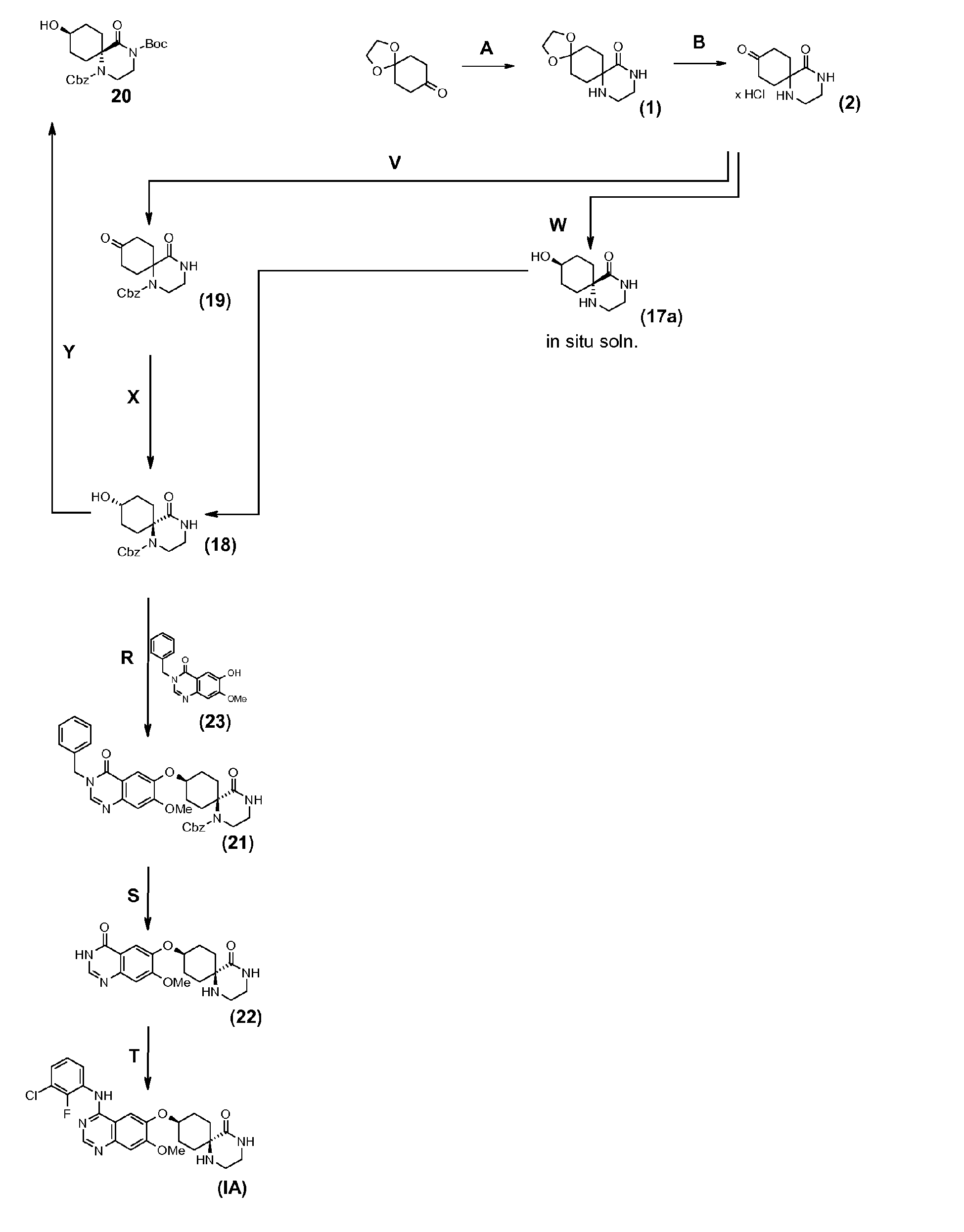

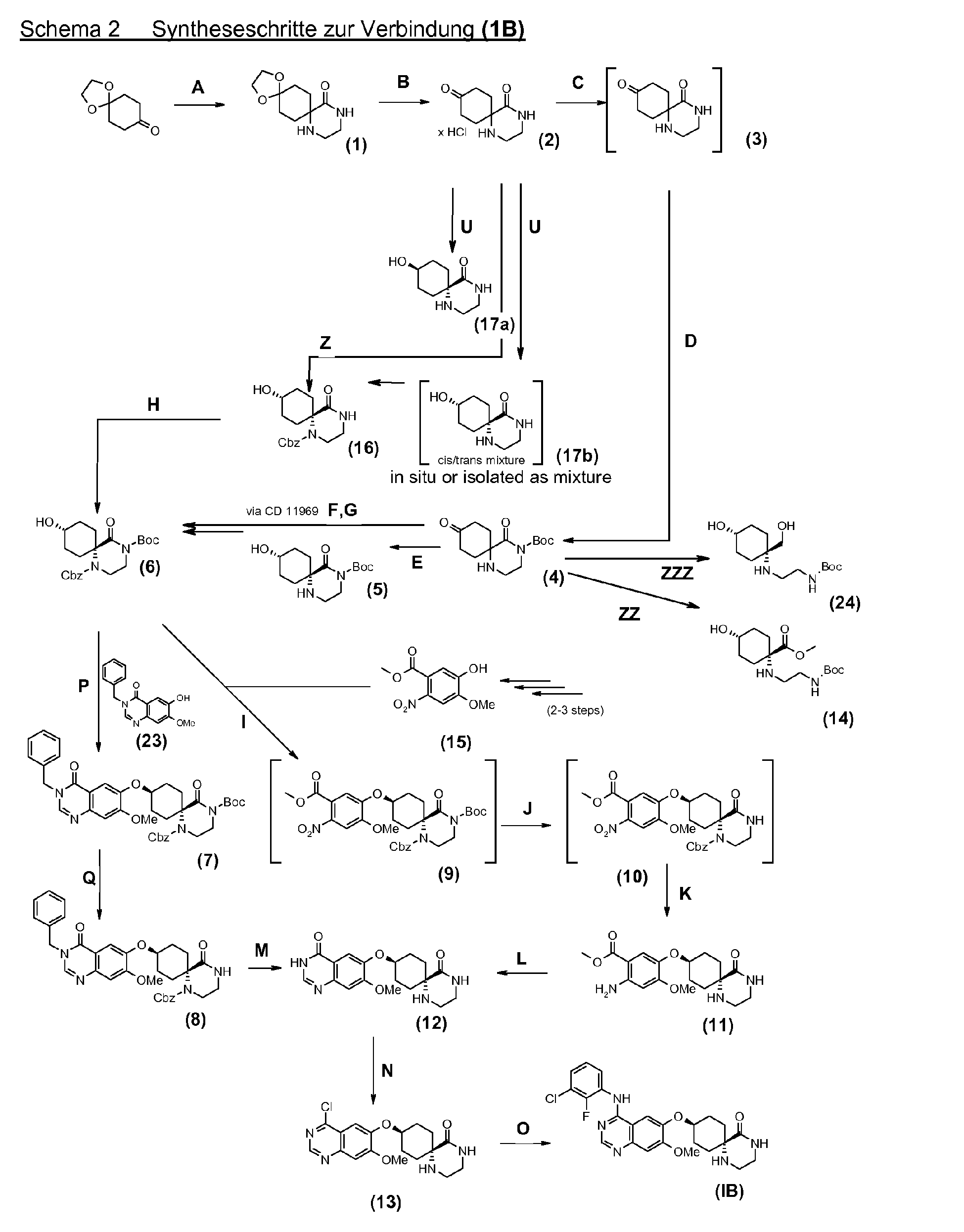

Die nachfolgenden Beispiele dienen der Illustration der exemplarisch durchgeführten Verfahren zur Herstellung der Verbindungen der Formen **(IA)** und **(IB).** Diese Beispiele sind als Erläuterung der Erfindung zu verstehen, ohne selbiges auf deren Gegenstand zu beschränken.

### Beispiel 1

1, 4-Dioxa-9,12-diaza-dispiro[4.2.5.2]pentadecan-13-one

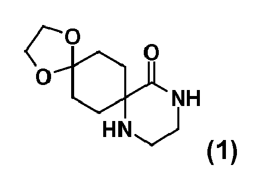

### Verfahrensschritt A

Zu einer auf -5°C abgekühlten Mischung aus 250 g 1,4.cyclohexandion-mono-ethylenketal, 18.2 g Benzyltriethylammoniumchlorid, 1.57 g Natriumcyanid in 1 I Dichlormethan werden 127.5 ml Ethylendiamin in 194 ml Chloroform zugetropft. Anschließend werden bei ca. -10 bis 0°C 407.5 ml 50%ige Natronlauge innerhalb der nächsten 9 h zugetropft. Nach 14.5 h bei -5 bis 25°C werden 500 ml konz. Salzsäure zugetropft. Der Niederschlag wird abfiltriert und zweimal mit je 500 ml Dichlormethan gewaschen. Das Filtrat wird phasengetrennt. Die wässrige Phase wird zweimal mit je 1 I Dichlormethan und einmal mit 500 ml Dichlormethan extrahiert. Die vereinigten organischen Phasen werden über Natriumsulfat getrocknet und im Vakuum eingeengt. Es werden 500 ml n-Butylacetat zugegeben und weiter soweit eingeengt, dass 820g Suspension übrig bleiben. Bei 50°C werden innerhalb von 20 min 3 I Methyl-tert-butylether zugegeben. Der Niederschlag wird abgesaugt und zweimal mit je 200 ml Methyl-tert-butylether gewaschen. Nach Trocknen erhält man 247 g Produkt.
Massenspektrum (ESI⁺): m/z = 227 [M+H]⁺

### Beispiel 2

1,4-Diaza-spiro[5.5]undecane-5,9-dione

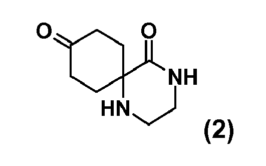

### Verfahrensschritt B

Zu 500 g 1,4-Dioxa-9,12-diaza-dispiro[4.2.5.2]pentadecan-13-one in 2.5 I Essigsäure werden 310 ml 10M HCl in Ethanol innerhalb von 45 min zugetropft. Nach 3 h bei 35-45°C werden innerhalb von 20 min 10 I Isopropanol zugetropft. Die Suspension wird auf 15°C abgekühlt und filtriert. Der Niederschlag wird zweimal mit je 1 I Isopropanol und zweimal mit je 1 I Methyl-tert-butylether gewaschen. Nach Trocknen des Feststoffs erhält man 386 g Produkt als Hydrochlorid.
Massenspektrum (ESI⁺): m/z = 183 [M+H]⁺

**Verfahrensschritt C** 380 g 1,4-Diaza-spiro[5.5]undecane-5,9-dione hydrochoride in 3.8 I Acetonitril werden innerhalb einer Stunde mit 320 ml 30%iger Natriummethylat-Lösung in Methanol versetzt. 18 g Natriumcarbonat werden zugegeben und der Ansatz für 18 h gerührt. Es werden 2 I Lösungsmittel abdestilliert und der Rückstand filtriert. Der Filterkuchen wird zweimal mit je 100 ml Acetonitril gewaschen und das Filtrat, welches das Produkt enthält, wird direkt in der nächsten Stufe weiter umgesetzt.

### Beispiel 3

5,9-Dioxo-1,4-diaza-spiro[5.5]undecane-4-carboxylic acid tert-butyl ester

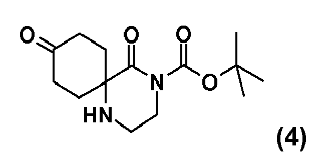

### Verfahrensschritt D

Zu der Lösung aus dem vorherigen Ansatz, die das 1,4-Diaza-spiro[5.5]undecane-5,9-dione enthält, werden 480 g Kaliumcarbonat und 10 g 4-(Dimethylamino)-pyridin zugefügt. 415 g Di-tert-butyldicarbonat in 415 ml Acetonitril werden innerhalb von 200 min zugetropft. Nach 18.5 h werden 10 g 4-(Dimethylamino)-pyridin und 100 g Di-tert-butyldicarbonat in 100 ml Acetonitril zugegeben. Nach 200 min werden 100 g Di-tert-butyldicarbonat in 100 ml Acetonitril zugegeben. Nach 90 min werden 50 g Di-tert-butyldicarbonat in 50 ml Acetonitril zugegeben. Nach 1 h werden 2 I Wasser zugegeben. Nach Phasentrennung wird die wässrige Phase mit 1 I Methyl-tert-butylether gewaschen. Die vereinigten organischen Phasen werden mit 1 I 10%iger Kaliumcarbonatlösung und 500 ml ges. Kochsalzlösung gewaschen. Die organische Phase wird im Vakuum eingeengt. Zur Suspension werden 1.5 I n-Butylacetat zugegeben und es wird erneut eingeengt. Es werden nochmals 2 I n-Butylacetat zugegeben und erneut eingeengt. Die zurückbleibende Suspension wird auf 55°C erwärmt und langsam mit 1 I Methyl-tert-butylether versetzt. Die Suspension wird auf 22°C abgekühlt. Der Niederschlag wird abfiltriert und mit 500 ml n-Butylacetat und 500 ml Methyl-tert-butylether gewaschen. Nach Trocknen des Feststoffs erhält man 296 g des Produkts.
Massenspektrum (ESI⁺): m/z = 283 [M+H]⁺

### Beispiel 4

(cis)-9-Hydroxy-5-oxo-1,4-diaza-spiro[5.5]undecane-4-carboxylic acid tert-butyl ester 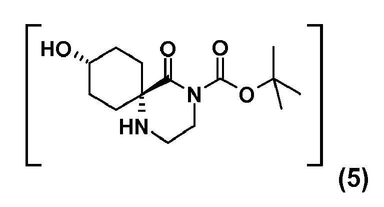 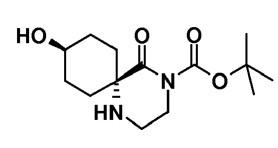 entsteht auch

### Verfahrensschritt E

Zu einer Mischung aus 159 g 5,9-Dioxo-1,4-diaza-spiro[5.5]undecane-4-carboxylic acid tert-butyl ester in 1140 ml Wasser werden bei 1°C innerhalb von 17 min 6.4 g Natriumborhydrid in 100 ml Wasser zugetropft. Der Tropftrichter wird mit 30 ml Wasser nachgespült. Nach 50 min werden 318 ml ges. Kaliumcarbonatlösung zugegeben und nach Rühren für 1 h bei 10°C wird der Niederschlag abgesaugt und zweimal mit je 200 ml 10%iger Kaliumcarbonatlösung gewaschen. Nach Trocknen wird der Niederschlag in 1.6 I Wasser für 4.5 h gerührt. Es werden 350 ml ges. Kaliumcarbonatlösung zugegeben und nach Rühren für 15 min wird der Niederschlag abgesaugt und mit 200 ml 10%iger Kaliumcarbonatlösung gewaschen. Nach Trocknen wird der Niederschlag in 500 ml Tetrahydrofuran für 20 min gerührt. Nach Filtration, Nachwaschen mit 200 ml Tetrahydrofuran und Eindampfen des Filtrats erhält man 65.5 g Produkt.
Massenspektrum (ESI⁺): m/z = 285 [M+H]⁺

### Verfahrensschritt F

Zu einer Lösung aus 113 g 5,9-Dioxo-1,4-diaza-spiro[5.5]undecane-4-carboxylic acid tert-butyl ester in 1150 ml THF und 25 ml Wasser werden bei 16°C innerhalb von 20 min 3.8 g Natriumborhydrid in 30 ml Wasser zugetropft. Nach 45 min werden 0.42 g Natriumborhydrid zugegeben. Nach 35 min werden 0.42 g Natriumborhydrid zugegeben. Nach weiteren 35 min werden 0.1 g Natriumborhydrid zugegeben. Nach 15 min werden 10 ml Aceton zugefügt und das Reaktionsgemisch wird mit zweimal mit je 500 ml ges. Kochsalzlösung gewaschen. Die organische Phase wird direkt im nächsten Versuch weiterverwendet.
Massenspektrum (ESI⁺): m/z = 285 [M+H]⁺

### Beispiel 5

(cis)-9-Hydroxy-5-oxo-1,4-diaza-spiro[5.5]undecane-1,4-dicarboxylic acid 1-benzyl ester 4-tert-butyl ester

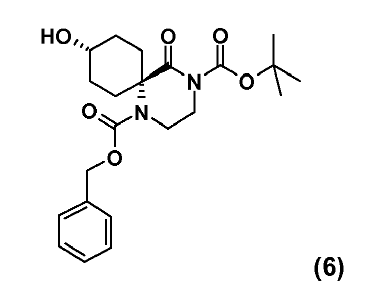

### Verfahrensschritt G

Zur Organischen Phase aus dem vorherigen Ansatz werden 112 ml ges. Kaliumcarbonatlösung zugegeben und anschließend werden 59 ml Chlorameisensäurebenzylester innerhalb von 20 min zugetropft. Nach 16 h werden 400 ml Wasser zugegeben und die Phasen getrennt. Die organische Phase wird mit 900 ml ges. Kaliumcarbonatlösung und zweimal mit je 450 ml ges. Kochsalzlösung gewaschen. Die organische Phase wird über Magnesiumsulfat getrocknet und anschließend eingeengt. Nachdem 1 I abdestilliert wurde werden 450 ml Methylcyclohexan zugegeben und weiter eingeengt. Es werden noch zweimal je 100 ml Methylcyclohexan zugegeben und weiter eingeengt, bis 168 Rohprodukt übrig bleiben. Das Rohprodukt wird dreimal aus Methanol/Wasser 1:1 umkristallisiert. Nach Trocknen erhält man 86 g Produkt.
Massenspektrum (ESI⁺): m/z = 419 [M+H]⁺

### Verfahrensschritt H :

Eine Mischung aus 500 mg (cis)-9-Hydroxy-5-oxo-1,4-diaza-spiro[5.5]undecane-1-carboxylic acid benzyl ester, 217 mg Kaliumcarbonat, 686 mg Di-tert-butyldicarbonat und 192 mg 4-(Dimethylamino)-pyridin in 10 ml Acetonitril werden für 4 h bei RT gerührt. Das Gemisch wird durch zweimalige Chromatographie an Kieselgel aufgereinigt und man erhält 420 mg Produkt.
Massenspektrum (ESI⁺): m/z = 419 [M+H]⁺

### Beispiel 6

5-Hydroxy-4-methoxy-2-nitro-benzoesäuremethylester

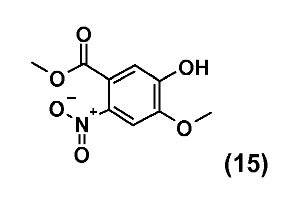

Eine Mischung aus 500 g 4,5-Dimethoxy-2-nitro-benzoesäuremethylester und 625 g Kaliumhydroxid in 2300 ml Wasser wird für 18.5 h auf 95 °C erhitzt. Nach Abkühlen wird klarfiltriert und das Filtrat mit 3 I Wasser verdünnt. Die Lösung wird mit 950 ml Essigsäure versetzt und nach 1 h wird der Niederschlag abfiltriert. Der Niederschlag wird in 3250 ml Ethylacetat suspendiert und anschließend werden 100 ml Wasser und 200 ml 12N Salzsäure zugegeben. Nach 1.5h werden die Phasen getrennt und die wässrige Phase wird mit 700 ml Ethylacetat extrahiert. Die vereinigten organischen Phasen werden über Magnesiumsulfat getrocknet und nach Filtration eingedampft. Es wird mit 200 ml Methylcyclohexan nachgedampft. Der Rückstand wird zusammen mit 1600 ml Methanol und 100 ml konz. Schwefelsäure für 16.5 h zum Rückfluss erhitzt. Der Ansatz wird eingeengt, bis die Kristallisation einsetzt. Es werden 1000 ml Wasser zugegeben und gerührt, bis eine homogene Suspension entstanden ist. Der Niederschlag wird abfiltriert, mit 500 ml Wasser gewaschen und in 1000 ml Wasser suspendiert. Nach 1.5 h Rühren wird der Niederschlag abfiltriert und mit 500 ml Wasser gewaschen. Nach Trocknen des Filterkuchens erhält man 364 g Produkt.
Massenspektrum (ESI⁻): m/z = 226 [M-H]⁺

### Beispiel 7

(trans)-9-(2-Methoxy-5-methoxycarbonyl-4-nitro-phenoxy)-5-oxo-1,4-diazaspiro[5.5]undecane-1,4-dicarboxylic acid 1-benzyl ester 4-tert-butyl ester

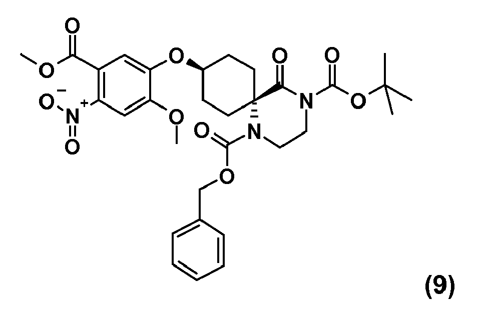

### Verfahrensschritt I

Zu einer Mischung aus 99 g (cis)-9-Hydroxy-5-oxo-1,4-diaza-spiro[5.5]undecane-1,4-dicarboxylic acid 1-benzyl ester 4-tert-butyl ester, 53.74 g 5-Hydroxy-4-methoxy-2-nitro-benzoic acid methyl ester **(15)** und 74.34 g Triphenylphosphin in 764 ml Dioxan werden bei RT 58.75 ml Azo-dicarbonsäure-diisopropylester innerhalb einer Stunde zugetropft. Nach 17 h werden 5 ml Azo-dicarbonsäure-diisopropylester zugegeben und der Ansatz für weitere 1.5 h gerührt. Die Mischung, die das Produkt enthält, wird ohne Aufreinigung direkt in der nächsten Stufe weiter umgesetzt.
Massenspektrum (ESI⁺): m/z = 645 [M+NH4]⁺

### Beispiel 8

(trans)-9-(2-Methoxy-5-methoxycarbonyl-4-nitro-phenoxy)-5-oxo-1,4-diazaspiro[5.5]undecane-1-carboxylic acid benzyl ester

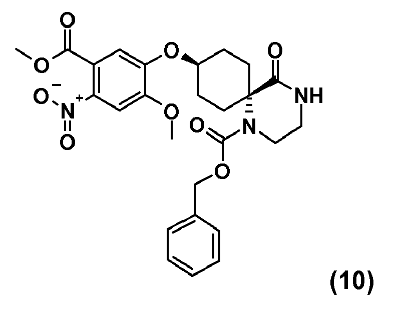

### Verfahrensschritt J

Zum vorherigen Ansatz, der den (trans)-9-(2-Methoxy-5-methoxycarbonyl-4-nitrophenoxy)-5-oxo-1,4-diaza-spiro[5.5]undecane-1,4-dicarboxylic acid 1-benzyl ester 4-tert-butyl ester enthält, werden 130 ml 4M HCl in Dioxan gegeben. Das Reaktionsgemisch wird auf 60°C erhitzt. Nach 2 h werden weitere 13 ml 4M HCl in Dioxan zugegeben. Die Reaktionslösung wird auf RT abgekühlt und mit 500 ml ges. Kaliumcarbonatlösung versetzt. Die organische Phase wird mit 500 ml ges. Kaliumcarbonat und 200 ml ges. Kochsalzlösung gewaschen. Die organische Phase, die das Produkt enthält, wird ohne Aufreinigung direkt in der nächsten Stufe weiter umgesetzt.
Massenspektrum (ESI⁺): m/z = 528 [M+H]⁺

### Beispiel 9

(trans)-2-Amino-4-methoxy-5-(5-oxo-1,4-diaza-spiro[5.5]undec-9-yloxy)-benzoic acid methyl ester

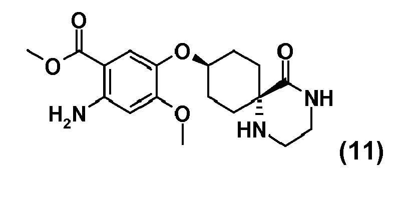

### Verfahrensschritt K

Zum vorherigen Ansatz, der den (trans)-9-(2-Methoxy-5-methoxycarbonyl-4-nitrophenoxy)-5-oxo-1,4-diazaspiro[5.5]undecane-1-carboxylic acid benzyl ester enthält, werden 12.4 g Pd (10%) auf Kohle und 500 ml Methanol zugefügt. Nach Hydrieren mit Wasserstoff für 1.5 h bei 3 bar wird der Ansatz bis auf 600 ml Restvolumen eingeengt. Der Ansatz wird mit 1.8 I Dioxan verdünnt und klarfiltriert. 59ml 4M HCl in Dioxan werden innerhalb von 45 min zugetropft und nach weiteren 30 min wird der Niederschlag abgesaugt und zweimal mit je 200 ml Dioxan gewaschen. Nach Trocknen des Feststoffs erhält man 98.6 g des Produkts als Hydrochlorid.
Massenspektrum (ESI⁺): m/z = 364 [M+H]⁺

### Beispiel 10

(trans)-9-(4-Hydroxy-7-methoxy-quinazolin-6-yloxy)-1,4-diaza-spiro[5.5]undecan-5-one

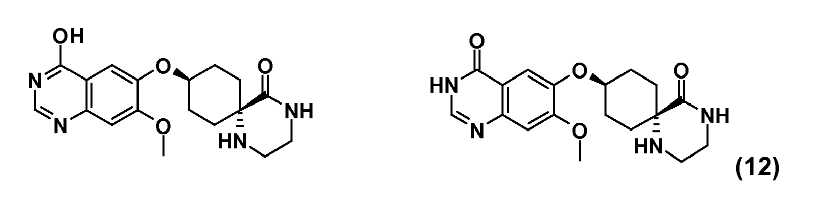

### Verfahrensschritt L

88 g (trans)-2-Amino-4-methoxy-5-(5-oxo-1,4-diaza-spiro[5.5]undec-9-yloxy)-benzoic acid methyl ester hydrochloride und 25 g Formamidinacetat in 1.8 L n-Propanol werden für 17h zum Rückfluss erhitzt. Anschließend wird auf 28°C abgekühlt und 4 h bei dieser Temperatur gerührt. Nach Abkühlen auf 14°C wird der Niederschlag abfiltriert und mit 200 ml kaltem n-Propanol gewaschen. Nach Trocknen des Feststoffs erhält man 44 g des Produkts als Hydrochlorid.
Massenspektrum (ESI⁺): m/z = 359 [M+H]⁺

### Verfahrensschritt M

Zu einer Mischung aus 1.7 g (trans)-9-(3-Benzyl-7-methoxy-4-oxo-3,4-dihydroquinazolin-6-yloxy)-5-oxo-1,4-diaza-spiro[5.5]undecane-1-carboxylic acid benzyl ester in 30 ml Essigsäure und 3 ml Wasser werden 300 mg Palladium (10%) auf Kohle zugefügt. Nach 22 h Hydrieren bei 70°C wird filtriert und die Lösung zur Trockne eingedampft, wobei 1.3g Produkt anfallen.
Massenspektrum (ESI⁺): m/z = 359 [M+H]⁺

### Beispiel 11

(trans)-9-(4-Chloro-7-methoxy-quinazolin-6-yloxy)-1,4-diaza-spiro[5.5]undecan-5-one

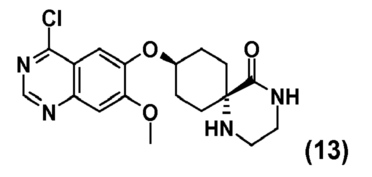

### Verfahrensschritt N

10 g (trans)-9-(4-Hydroxy-7-methoxy-quinazolin-6-yloxy)-1,4-diazaspiro[5.5]undecan-5-one hydrochloride und 12 g Triphenylphosphin werden in 450 ml Dioxan suspendiert. Anschließend werden 250ml Lösungsmittel abdestilliert und 6.45 g N-Chlorsuccinimid in 100 ml Acetonitril bei 41°C zugetropft. Die Reaktionsmischung wird zum Rückfluss erhitzt. Nach 100 min wird der Ansatz auf 29°C abgekühlt und 150 ml Methyltetrahydrofuran zugegeben. Der Niederschlag wird abfiltriert und dreimal mit je 50 ml Methyltetrahydrofuran gewaschen. Nach Trocknen bei 30°C erhält man 12 g eines dunkel gefärbten Feststoffs, der das Produkt als Hydrochlorid enthält, und der ohne Aufreinigung in der nächsten Stufe weiter umgesetzt wird.
Massenspektrum (ESI⁺): m/z = 377 [M+H]⁺

### Beispiel 12

(trans)-9-[4-(3-Chloro-2-fluoro-phenylamino)-7-methoxy-quinazolin-6-yloxy]-1,4-diaza-spiro[5.5]undecan-5-one

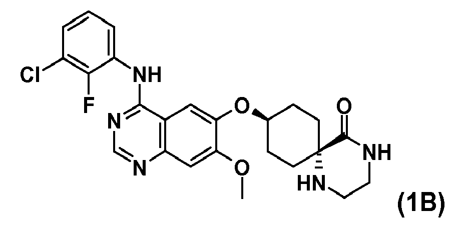

### Verfahrensschritt O

12g des unreinen (trans)-9-(4-Chloro-7-methoxy-quinazolin-6-yloxy)-1,4-diazaspiro[5.5]undecan-5-one hydrochlorides aus vorheriger Stufe werden bei RT innerhalb von 90 min portionsweise zu einer Lösung von 3.9 g 3-Chlor-2-fluoranilin in 60 ml 2N Salzsäure gegeben. Die Suspension wird für 60 min auf 40°C erwärmt. Anschließend werden 60 ml Toluol zugegeben und der Ansatz auf RT abgekühlt. Nach 50 min wird filtriert und der Niederschlag mit 50 ml Toluol und 50ml ges. NaCl-Lösung gewaschen. Nach Trocknen bei 40°C erhält man 10 g eines Feststoffs, der das Produkt enthält. Das Produkt wird durch basische Chromatographie an Kieselgel aufgereinigt.
Massenspektrum (ESI⁺): m/z = 486 [M+H]⁺

### Beispiel 13

(trans)-9-(3-Benzyl-7-methoxy-4-oxo-3,4-dihydro-quinazolin-6-yloxy)-5-oxo-1,4-diazaspiro[5.5]undecane-1,4-dicarboxylic acid 1-benzyl ester 4-tert-butyl ester

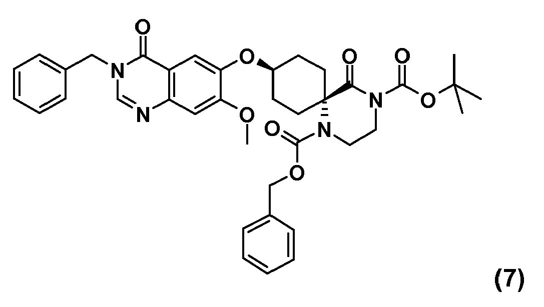

### Verfahrensschritt P

Zu einer Suspension aus 1.3 g 3-Benzyl-6-hydroxy-7-methoxy-3H-quinazolin-4-one, 2g (cis)-9-Hydroxy-5-oxo-1,4-diaza-spiro[5.5]undecane-1,4-dicarboxylic acid 1-benzyl ester 4-tert-butyl ester und 1.8 g Triphenylphosphin in 10 ml N-Methyl-2-pyrrolidon werden 1.36 ml Azo-dicarbonsäure-diisopropylester innerhalb von 90 min zugetropft. Der Ansatz wird für 4 h gerührt. Der Ansatz, der das Produkt enthält, wird direkt in der nächsten Stufe weiterverwendet.
Massenspektrum (ESI⁺): m/z = 683 [M+H]⁺

### Beispiel 14

(trans)-9-(3-Benzyl-7-methoxy-4-oxo-3,4-dihydro-quinazolin-6-yloxy)-5-oxo-1,4-diazaspiro[5.5]undecane-1-carboxylic acid benzyl ester

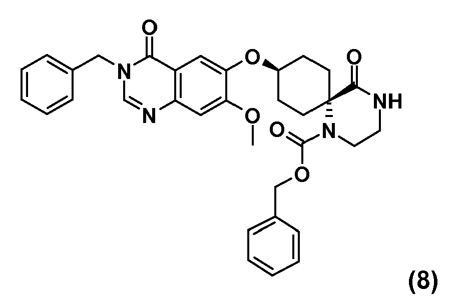

### Verfahrensschritt Q

Zum Ansatz aus vorheriger Stufe, der den (trans)-9-(3-Benzyl-7-methoxy-4-oxo-3,4-dihydro-quinazolin-6-yloxy)-5-oxo-1,4-diaza-spiro[5.5]undecane-1,4-dicarboxylic acid 1-benzyl ester 4-tert-butyl ester enthält, werden 2.5 ml 4 M HCl in Dioxan zugegeben. Nach 19 h werden 2 ml 4 M HCl in Dioxan zugegeben und der Ansatz auf 40°C erwärmt. Nach 3 h wird die Temperatur auf 60°C erhöht, der Ansatz mit 60 ml Dioxan verdünnt und 10 ml 4 M HCl in Dioxan zugegeben. Nach 16 h wird der Ansatz im Vakuum eingeengt und der Rückstand in 50 ml Dichlormethan aufgenommen. Nach dreimaligem Waschen mit jeweils 50 ml Wasser wird die organische Phase eingedampft. Der Rückstand wird chromatographisch an Kieselgel aufgereinigt. Die entsprechenden Fraktionen werden eingedampft und der Rückstand mit 150 ml Ethylacetat ausgekocht. Nach Isolation und Trocknen des Niederschlags erhält man 2.1 g Produkt.
Massenspektrum (ESI⁺): m/z = 583 [M+H]⁺

### Beispiel 15

(cis)-9-(3-Benzyl-7-methoxy-4-oxo-3,4-dihydro-quinazolin-6-yloxy)-5-oxo-1,4-diazaspiro[5.5]undecane-1-carboxylic acid benzyl ester

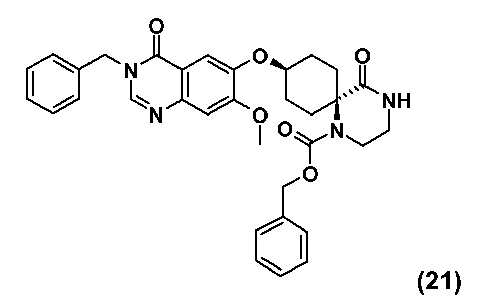

### Verfahrensschritt R

Zu einer Mischung aus 2 g 3-Benzyl-6-hydroxy-7-methoxy-3H-quinazolin-4-one, 2.37g (trans)-9-Hydroxy-5-oxo-1,4-diaza-spiro[5.5]undecane-1-carboxylic acid benzyl ester und 2.79 g Triphenylphosphin in 20 ml N-Methyl-2-pyrrolidon werden unter Kühlung 2.1 ml Azo-dicarbonsäure-diisopropylester zugetropft. Nach 20 min werden 20 ml N-Methyl-2-pyrrolidon zugefügt und der Ansatz wird für 4 h gerührt. Der Niederschlag wird bei 0°C abgesaugt und mit 50 ml Methyl-tert-butylether gewaschen. Nach Trocken erhält man 3.3g Produkt, das noch N-Methyl-2-pyrrolidon enthält.
Massenspektrum (ESI⁺): m/z = 583 [M+H]⁺

### Beispiel 16

(cis)-9-(4-Hydroxy-7-methoxy-quinazolin-6-yloxy)-1,4-diaza-spiro[5.5]undecan-5-one

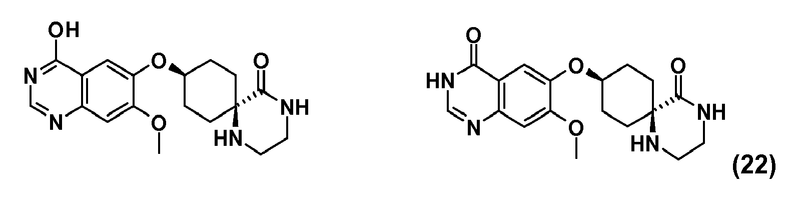

### Verfahrensschritt S

Zu einer Mischung aus 1.7 g (cis)-9-(3-Benzyl-7-methoxy-4-oxo-3,4-dihydroquinazolin-6-yloxy)-5-oxo-1,4-diaza-spiro[5.5]undecane-1-carboxylic acid benzyl ester in 30 ml Ethanol und 10 ml 1 M Salzsäure werden 300 mg Palladium (10%) auf Kohle zugefügt. Nach 25 h Hydrieren bei 80°C wird filtriert und die Lösung zur Trockne eingedampft, wobei 1.4g Rohprodukt anfallen. Das Rohprodukt wird mit 100 ml Ethanol ausgekocht und nach Filtration wird das Filtrat eingeengt. Der Rückstand wird in 50 ml Acetonitril suspendiert und nach Zugabe von 1 g Kaliumcarbonat für 23 h gerührt. Der Ansatz wird eingeengt und nach Zugabe von 20 ml Dichlormethan und 4 ml Methanol chromatographisch an Kieselgel aufgereinigt. 500 mg Produkt werden erhalten.
Massenspektrum (ESI⁺): m/z = 359 [M+H]⁺

### Beispiel 17

(cis)-9-[4-(3-Chloro-2-fluoro-phenylamino)-7-methoxy-quinazolin-6-yloxy]-1,4-diazaspiro[5.5]undecan-5-one

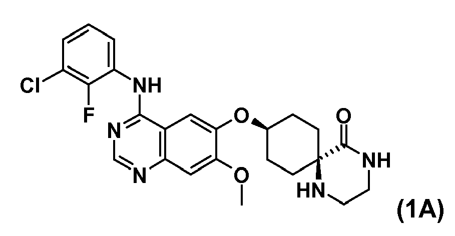

### Verfahrensschritt T

Zu einer Mischung vom 100 mg 7-Methoxy-6-(5-oxo-1,4-diaza-spiro[5.5]undec-9-yloxy)-3H-quinazolin-4-one und 0.23 ml Triethylamin in 5 ml Acetonitril werden 0.13 ml Phosphoroxytrichlorid zugefügt. Nach 1 h werden 0.04 ml 3-Chlor-2-Fluoranilin zugegeben. Nach 18 h wird 1 ml Wasser zugefügt und die das Gemisch bsi auf 2 ml Volumen eingeengt. Nach Aufreinigung mittels präparativer HPLC erhält man 95 mg Produkt.
Massenspektrum (ESI⁺): m/z = 486 [M+H]⁺

### Beispiel 18

(trans)-9-Hydroxy-1,4-diaza-spiro[5.5]undecan-5-one hydrochloride (cis)-9-Hydroxy-1,4-diaza-spiro[5.5]undecan-5-one hydrochloride

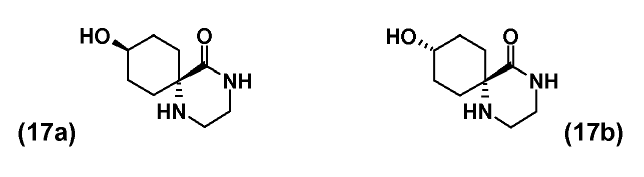

### Verfahrensschritt U

Zu einer Mischung aus 500 mg 1,4-Diaza-spiro[5.5]undecane-5,9-dione hydrochloride in 5 ml Wasser werden 50 mg Platindioxid zugefügt. Nach 3 h Hydrieren wird filtriert und die Lösung zur Trockne eingedampft. Es wird zweimal mit je 50 ml n-Propanol nachgedampft und zurück bleiben 500mg eines trans-/cis-Gemisches von 9-Hydroxy-1,4-diaza-spiro[5.5]undecan-5-one hydrochloride.
Massenspektrum (ESI⁺): m/z = 185 [M+H]⁺

### Beispiel 19

5,9-Dioxo-1,4-diaza-spiro[5.5]undecane-1-carboxylic acid benzyl ester

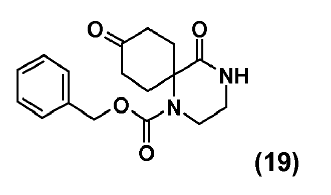

### Verfahrensschritt V

Zu einer Mischung aus 20g 1,4-Diaza-spiro[5.5]undecane-5,9-dione hydrochloride in 100 ml Tetrahydrofuran und 82 ml 50%iger Kaliumcarbonatlösung werden unter Kühlung 14.4 ml Chlorameisensäurebenzylester zugegeben. Nach 2.5 h werden 250 ml Wasser zugefügt und der Niederschlag abfiltriert. Nach Waschen mit 200 ml Wasser und 200 ml Methyl-tert-butylether und Trocknen erhält man 24.3 g Produkt.
Massenspektrum (ESI⁺): m/z = 317 [M+H]⁺

### Beispiel 20

(trans)-9-Hydroxy-5-oxo-1,4-diaza-spiro[5.5]undecane-1-carboxylic acid benzyl ester

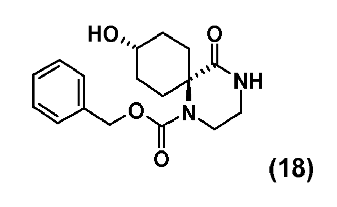

### Verfahrensschritt W

Zu einer Mischung aus 5 g 1,4-Diaza-spiro[5.5]undecane-5,9-dione hydrochloride in 20 ml Wasser werden 50 mg Platindioxid zugefügt. Nach 22 h Hydrieren werden 25 mg Platindioxid zugefügt. Nach 26 h Hydrieren wird filtriert und das Filtrat mit 35 g Kaliumcarbonat und 25 ml Tetrahydrofuran versetzt. 3.43 ml Chlorameisensäurebenzylester werden zugegeben und der Ansatz für 6 d gerührt. 25 g Kaliumcarbonat werden zugegeben und der Ansatz für 4 d gerührt. 3.5 ml Chlorameisensäurebenzylester werden zugegeben. Nach 20 h werden 200 ml Wasser zugefügt und nach 1 h nachrühren wird der Niederschlag abgesaugt und mit 100 ml Methyl-tert-butylether gewaschen. Man erhält 3.4 g Feststoff, der hauptsächlich aus dem Produkt besteht.
Massenspektrum (ESI⁺): m/z = 319 [M+H]⁺

### Verfahrensschritt X

Zu 20 g 5,9-Dioxo-1,4-diaza-spiro[5.5]undecane-1-carboxylic acid benzyl ester in 100 ml Tetrahydrofuran, 100 ml Ethanol, 80 ml Wasser und 20 ml 0.1 N Natronlauge werden 7.2 g Natriumborhydrid gegeben. Nach 16.5 h Rühren bei RT und 1h bei 60°C werden unter Eiskühlung 80 ml 2M Salzsäure und 200 ml Wasser zugetropft. Nach 2 h wird der Niederschlag abgesaugt und mit 200 ml Wasser gewaschen. Nach Trocknen des Niederschlags und Chromatographische Reinigung an Kieselgel werden 8 g Produkt isoliert.
Massenspektrum (ESI⁺): m/z = 319 [M+H]⁺

### Beispiel 21

(trans)-9-Hydroxy-5-oxo-1,4-diaza-spiro[5.5]undecane-1,4-dicarboxylic acid 1-benzyl ester 4-tert-butyl ester

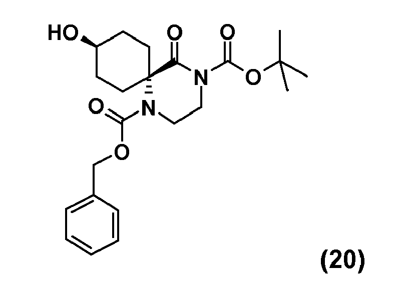

### Verfahrensschritt Y

Eine Mischung aus 200 mg (trans)-9-Hydroxy-5-oxo-1,4-diaza-spiro[5.5]undecane-1-carboxylic acid benzyl ester, 87 mg Kaliumcarbonat, 274 mg Di-tert-butyldicarbonat und 76 mg 4-(Dimethylamino)-pyridin in 5 ml Acetonitril werden für 2 h bei RT gerührt. Das Gemisch wird durch präparative HPLC aufgereinigt und man erhält 100 mg Produkt.
Massenspektrum (ESI⁺): m/z = 419 [M+H]⁺

### Beispiel 22

(cis)-9-Hydroxy-5-oxo-1,4-diaza-spiro[5.5]undecane-1-carboxylic acid benzyl ester

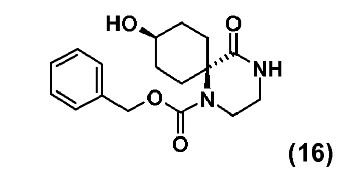

### Verfahrensschritt Z

Zu einer Lösung von 75 g ,4-Diaza-spiro[5.5]undecane-5,9-dione hydrochoride in 350 ml 1 M Natronlauge werden bei RT portionsweise 14.3 g Natriumborhydrid gegeben. Nach 35 min werden unter Kühlung innerhalb von 30 min 60 ml konz. Salzsäure zugetropft. Es werden 390 g Kaliumcarbonat zugefügt. Nach Zugabe von 300 ml Tetrahydrofuran und 67 ml Chlorameisensäurebenzylester wird der Ansatz für 1.5 h auf 48°C erhitzt. Es werden 900 ml Methyl-tert-butylether zugegeben und nach abkühlen auf 22°C 1.6 I Wasser zugefügt. Nach 1h Rühren wird die Suspension abgesaugt und der Filterkuchen mit 500 ml Wasser und 1 I Methyl-tert-butylether gewaschen. Nach Trocknen des Filterkuchens erhält man 77 g Produkt, das hauptsächlich aus dem cis-Isomer besteht.
Massenspektrum (ESI⁺): m/z = 319 [M+H]⁺

### Beispiel 23

(cis)-1-(2-tert-Butoxycarbonylamino-ethylamino)-4-hydroxy-cyclohexanecarboxylic acid methyl ester

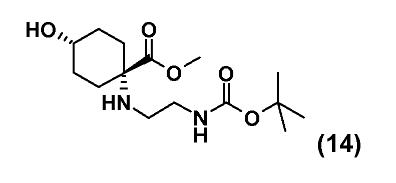

### Verfahrensschritt ZZ

Zu einer Lösung aus 500 mg 5,9-Dioxo-1,4-diaza-spiro[5.5]undecane-4-carboxylic acid tert-butyl ester in 5 ml Methanol werden 16.7 mg Natriumborhydrid zugefügt. Nach 4 h wird der Ansatz eingedampft und mit Tetrahydrofuran nachgedampft. Der Rückstand enthält das Produkt.
Massenspektrum (ESI⁺): m/z = 317 [M+H]⁺

### Beispiel 24

(**cis**)-[2-(4-Hydroxy-1-hydroxymethyl-cyclohexylamino)-ethyl]-carbamic acid tert-butyl ester

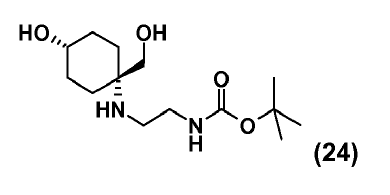

### Verfahrensschritt ZZZ

Zu einer Mischung aus 1 g 5,9-Dioxo-1,4-diaza-spiro[5.5]undecane-4-carboxylic acid tert-butyl ester in 10 ml 1 M Kaliumcarbonatlösung werden unter Kühlung 161 mg Natriumborhydrid zugefügt. Nach 14.5 h bei 50°C werden 10 ml Ethylacetat zugegeben und nach Phasentrennung die organische Phase eingeengt. Nach chromatographischer Aufreinigung des Rückstands an Kieselgel werden 580 mg eines Gemisches isoliert, das das Produkt enthält.
Massenspektrum (ESI⁺): m/z = 289 [M+H]⁺

## Patentansprüche

1. Verfahren zur stereoselektiven Herstellung der Verbindung der Formel **(IA)** 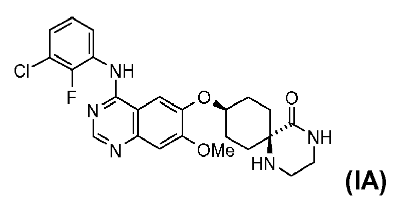 gegebenenfalls in Form ihrer Tautomeren, sowie gegebenenfalls ihrer pharmakologisch unbedenklichen Säureadditionssalze,
**dadurch gekennzeichnet, dass** das Verfahren die Reaktionsschritte **(A), (B), (V), (X), (R), (S)** und **(T)** umfasst, wobei
**(A)** die Umsetzung von 1,4.cyclohexandion-mono-ethylenketal zu einer Verbindung der Formel **(1)** 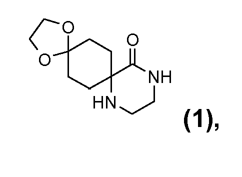
**(C)** die Umsetzung einer Verbindung der Formel **(1)** zu der Verbindung der Formel **(2)** 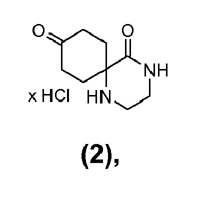
**(V)** die Umsetzung einer Verbindung der Formel **(2)** mit Chlorameisensäurebenzylester zu der Verbindung der Formel **(19)** 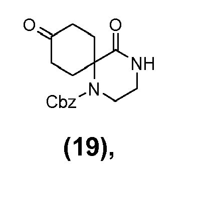
**(X)** die Umsetzung einer Verbindung der Formel **(19)** zu der Verbindung der Formel **(18)** 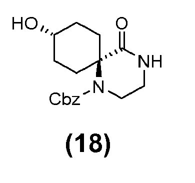
**(R)** die Umsetzung einer Verbindung der Formel **(18)** mit einer Verbindung der Formel **(23)** 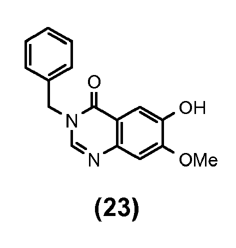 zu einer Verbindung der Formel **(21)** 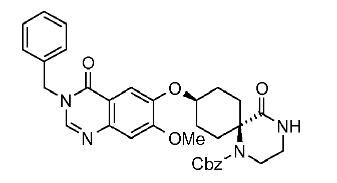 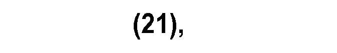
**(S)** die Abspaltung des Benzylrestes der Verbindung der Formel **(21)** zu einer Verbindung der Formel **(22)** 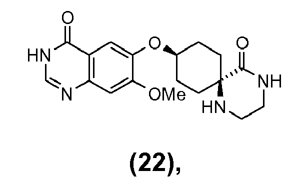 und
**(T)** die Chlorierung der Verbindung der Formel **(22)** und anschließende Umsetzung mit 3-Chlor-2-Fluoranilin
bedeutet,
wobei die Schritte **(A)** bis **(T)** in der genannten Reihenfolge nacheinander erfolgen.

2. Verfahren nach Anspruch 1 zur stereoselektiven Herstellung von Verbindungen der Formel **(IA), dadurch gekennzeichnet, dass** das Verfahren aus den Verfahrensschritten **(R ), (S)** und **(T)** besteht.

3. Verfahren nach Anspruch 1 zur stereoselektiven Herstellung einer Verbindung der Formel **(18), dadurch gekennzeichnet, dass** das Verfahren aus den Verfahrensschritten **(A), (B), (V)** und **(X)** besteht.

4. Verfahren zur stereoselektiven Herstellung der Verbindung der Formel **(IB)** 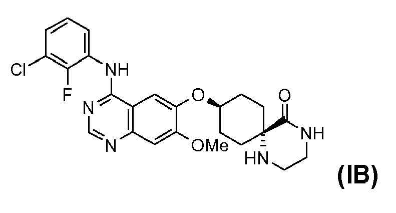 gegebenenfalls in Form ihrer Tautomeren, sowie gegebenenfalls ihrer pharmakologisch unbedenklichen Säureadditionssalze,
**dadurch gekennzeichnet, dass** das Verfahren die Reaktionsschritte **(A), (B), (Z), (H), (P), (Q), (M), (N)** und **(O)** umfasst, wobei
**(A)** die Umsetzung von 1,4.Cyclohexandion-mono-ethylenketal zu einer Verbindung der Formel **(1)** 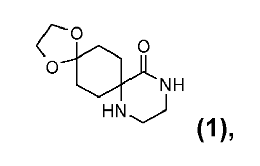
**(C)** die Umsetzung einer Verbindung der Formel **(1)** zu der Verbindung der Formel **(2)** 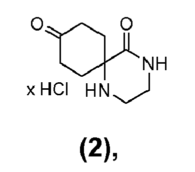
**(Z)** die Umsetzung einer Verbindung der Formel **(2)** zu der Verbindung der Formel **(16)** 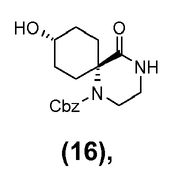
**(H)** die Umsetzung einer Verbindung der Formel **(16)** zu der Verbindung der Formel **(6)** 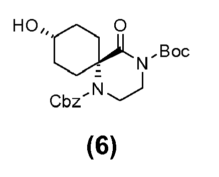
**(P)** die Umsetzung einer Verbindung der Formel **(6)** mit einer Verbindung der Formel **(23)** 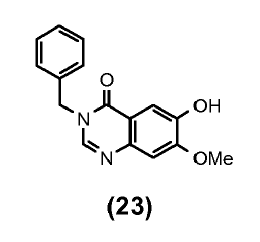 zu einer Verbindung der Formel **(7)** 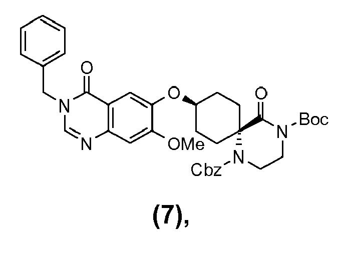
**(Q +M)** die Abspaltung dr Schutzgruppen der Verbindung der Formel **(7)** zu einer Verbindung der Formel **(12)** 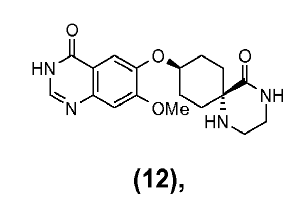 und
**(N + O)** die Chlorierung der Verbindung der Formel **(12)** und anschließende Umsetzung mit 3-Chlor-2-Fluoranilin
bedeutet,
wobei die Schritte **(A)** bis **(O)** in der genannten Reihenfolge nacheinander erfolgen.

5. Verfahren gemäß Anspruch 4 zur stereoselektiven Herstellung einer Verbindung der Formel **(1B), dadurch gekennzeichnet, dass** in dem Verfahren die Verfahrensschritte **[(Z), (H)]** durch die Verfahrensschritte **[(C ), (D), (E), (F)]** ersetzt werden, wobei
**(C)** die Umsetzung einer Verbindung der Formel **(2)** zu der Verbindung der Formel **(3)** 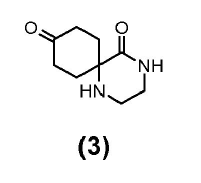
**(D)** die Umsetzung einer Verbindung der Formel **(3)** zu der Verbindung der Formel **(4)** 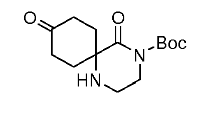 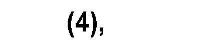
**(E)** die Umsetzung einer Verbindung der Formel **(4)** zu der Verbindung der Formel **(5)** 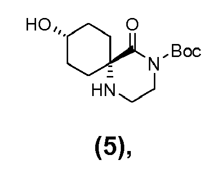 und
**(F)** die Umsetzung einer Verbindung der Formel **(5)** zu der Verbindung der Formel **(6)** 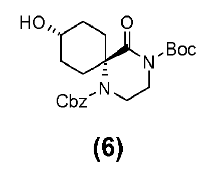 bedeuten,
wobei die Schritte **(C)** bis **(F)** in der genannten Reihenfolge nacheinander erfolgen.

6. Verfahren gemäß Anspruch 4 oder 5 zur stereoselektiven Herstellung einer Verbindung der Formel **(1B), dadurch gekennzeichnet, dass** in dem Verfahren die Verfahrensschritte **[(P), (Q), (M)]** durch die Verfahrensschritte **[(I** ), **(J), (K), (L)]** ersetzt werden, wobei
**(I)** die Umsetzung einer Verbindung der Formel **(6)** mit einer Verbindung der Formel **(15)** 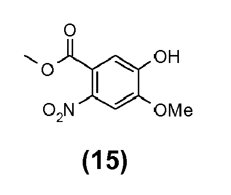 zu der Verbindung der Formel **(9)** 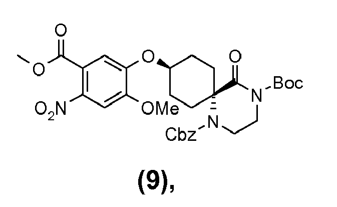
**(J + K)** die Abspaltung der Schutzgruppen und hydrogenolytische Reduktion einer Verbindung der Formel **(9)** zu der Verbindung der Formel **(11)** 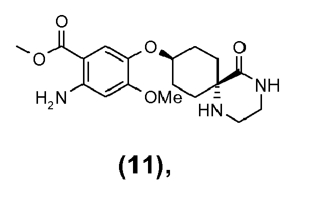 und
**(L)** die Umsetzung einer Verbindung der Formel **(11)** zu der Verbindung der Formel **(12)** 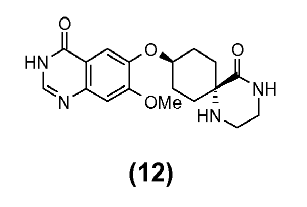 bedeuten,
wobei die Schritte **(I)** bis **(L)** in der genannten Reihenfolge nacheinander erfolgen.

7. Verbindung gemäß Anspruch 1 der Formel 1A, sowie deren physiologisch verträgliche Salze mit anorganischen oder organischen Säuren und Basen.

8. Verbindung gemäß Anspruch 4 der Formel **(1B)** sowie deren physiologisch verträgliche Salze mit anorganischen oder organischen Säuren und Basen.

9. Verbindung gemäß Anspruch 1 der Formel **(18),** sowie deren physiologisch verträgliche Salze mit anorganischen oder organischen Säuren und Basen.

10. Verbindung gemäß Anspruch 1 der Formel **(22),** sowie deren physiologisch verträgliche Salze mit anorganischen oder organischen Säuren und Basen.

11. Verbindung gemäß Anspruch 4 der Formel **(13),** sowie deren physiologisch verträgliche Salze mit anorganischen oder organischen Säuren und Basen.

12. Verbindung gemäß Anspruch 4 der Formel **(16),** sowie deren physiologisch verträgliche Salze mit anorganischen oder organischen Säuren und Basen.

13. Verbindung gemäß Anspruch 4 der Formel **(6),** sowie deren physiologisch verträgliche Salze mit anorganischen oder organischen Säuren und Basen.
